# EUROPEAN PATENT APPLICATION

(11) **EP 1 541 495 A1**
(43) Date of publication of application: **15.06.2005**
(21) Application number: 04257643.9
(22) Date of filing: 09.12.2004
(51) Int. Cl.: B65D 81/26, A61L 9/12

(54) **Packaging for eliminating off-odors**

(30) Priority: 12.12.2003 US 734426
(71) Applicant: INTERNATIONAL FLAVORS & FRAGRANCES INC., New York New York 10019 (US); FRITO-LAY NORTH AMERICA, INC., Plano, TX 75024-4099 (US)
(72) Inventor: Brown, Martha Jo Meadows, Grapevine, Texas 76051 (US); Licker, Jonathan Louis, McKinney, Texas 75071 (US); Zbuchalski, Michael R., Plano, Texas 75093 (US)
(74) Representative: Mercer, Christopher Paul

(57) **Abstract**

Packaging materials and packages that transfer off-odor eliminating compounds (103) to foods and other products via the vapor phase are disclosed. The present invention provides a source of desired off-odor eliminating compound, a suitable environment for vapor transfer, and an appropriate product substrate to which an off-odor eliminating compound is added. Examples of the source of off-odor eliminating compound can be a packaging film (102) containing off-odor eliminating compound, a sachet (300) of absorbed flavor material, a tape or label (310) applied to the inside of a package, and a flavor diffusing granule, or alternatively an active system for delivering a vapor to the environment. Alternatively, the off-odor eliminating compounds can be applied topically, directly to the food or other product. In one preferred embodiment, a sulphur scavenging material is used as the off-odor eliminating compound. Materials that are preferably treated using the present invention include snacks, confections, baked goods, fresh plant materials, cereals and beverages, as well as non-food products.

## Description

The present invention relates to packaging food and other consumer products and to packaging materials, and more particularly to polymeric packaging materials.

### BACKGROUND OF THE INVENTION

A wide variety of foods and consumer products rely on flavors and fragrances to enhance their appeal. Fragrances in particular play a large role in creating an appeal or attraction toward a particular product, cooperating with the senses of sight and touch to create an overall sensory impression. A fragrance is any molecule that diffuses via vaporization into the atmosphere (under local conditions of temperature and pressure) and subsequently activates a specific receptor in the nasal cavity. Similarly, volatile flavors activate a specific receptor in the nasal cavity but enter retronasally after being placed in the mouth. A non-volatile flavor, on the other hand, is conventionally referred to as a "taste," e.g., salt, sugar monosodium glutamate and non-volatile acids, and operates via activation of specific receptors within the mouth after they are diffused in the saliva. Therefore, in terms of the present invention both volatile flavors and fragrances can be classified as "fragrances." Although fragrances are extremely important for all forms of food and drink, they also play a role in the desirability of other consumer products that are not ingested.

The sensations relating to smell and taste are complex. Often the level of fragrances needed in a product to achieve significant human response is very low. This is particularly true for a fragrance that has a high tendency to diffuse under normal atmospheric conditions (high vapor pressure) and has a low odor threshold (high odor activity). Due to their nature, however, these are often lost during the stress of processing and/or distribution that occur during the chain of production. Typically, attempts have been made to compensate for these losses by over-dosing the desired flavor components, or attempting to protect the flavor component via some form of encapsulation. For example, U.S. Patent No. 6,299,917-Appleby discloses impregnating liquid smoke flavoring into cellulose for use as a meat casing. Although these methods can be functional in some cases they often result in unbalanced profiles due to the uncertain nature of the flavor loss, particularly relative to the change over time during the shelf life. Moreover, techniques such as adding fragrance to the product or encapsulation of the product add undesirably to its cost. Additionally, it is often difficult to maintain the appearance, texture, integrity or overall state of the product using these techniques. In the realm of foodstuffs, fresh fruits and vegetables are prime examples of items that are difficult to "treat" with additional flavor or aroma. While an injection process, for example, might be effective, discoloration or other degradation of the fruit itself would likely result.

A dose of scented substance can be applied to a package so it diffuses into the headspace without contacting the product. For example, U.S. Patent No. 4,564,535-Altenschopfer, et al. discloses scenting cardboard packaging materials applying scented pastes to the packaging material by spraying, using a spreading device, or painting. The Altenschopfer, et al. patent discusses the difficulty of scenting the product itself, and discloses that a gel containing a perfume should be applied to the upper surface of a cardboard box to avoid contact with the product. Similarly, U.S. Patent No. 6,394,264-Rivello is directed to a cap that contains a "dose" of fragrance that is held within a semi-permeable barrier so it diffuses into the headspace above the liquid in a bleach or detergent bottle, but avoids contact with the actual product. Both references discuss the difficult problem of adding a scent to bleaches and detergents, due to the reactive nature of bleach.

Others have focused on providing perfume samples in a manner that preserves them, yet permits them to diffuse into the air or be applied to the skin when the packaging is opened. For example, U.S. Patent 5,885,701-Berman et al.-a colloidal suspension odorant composition comprised of a colloidal material and a liquid fragrance component is provided in a package comprised of a multiple layer top substrate and a multiple layer bottom. Similarly, U.S. Patent No. 4,880,690-Szycher describes a system using a polyurethane layer in connection with fragrance oil to assist in the emission of fragrance on a perfume patch after the sealed package is opened.

Additionally, many products develop an off-odor (or "malodor") during storage that detracts from product enjoyment. In particular, various foodstuffs develop off-odors due to the complex interactions of the food, preservatives and spices with each other, and with the oxygen and other gases in the package. As noted above, the sense of smell is powerfully related to the enjoyment of food, and therefore, it is in as important to reduce or eliminate off-odors as it is to enhance desirable fragrances and flavors. Sulphur compounds in particular are known to affect perceptions of odor and taste hedonics. Various sulfides and sulfur mercaptans have been specifically identified, e.g., methyl sulfide is the odor produced by a rotten egg.

Others have attempted to control off-odor in products contained in packages. For example, U.S. Patent No. 6,218,013-Wood et al. discloses a barrier material comprising a thermoplastic and a compatible cyclodextrin derivative in which a permeant is entrapped by the cyclodextrin compound. The permeant does not pass through the film into the enclosure or container to cause off odors. Additionally, there has been extensive development of oxygen scavenging technology in both flexible and rigid packaging application. For one example, U.S. Patent No. 6,607,795-Yang, et al. discloses oxygen scavenging compositions that, scavenge (i.e., react irreversibly) with oxygen from either the inside of a product package or that have permeated across the package from the exterior. Others have suggested that scavenging compounds diminish the desired fragrance and should therefore be inhibited. For example, U.S. Patent No. 6,495,097-Streit, et al. discloses lessening the undesirable scavenging of fragrances and flavors in products incorporating undecylenic acid by pre-mixing the undecylenic acid and/or its derivatives with fragrances and flavor agents in a medium.

None of these prior art packages address the problems outlined above. Commonly assigned U.S. Patent Application Number 10/202,958 filed July 25, 2003 and continuation in part application U.S. Serial Number 10/441,574 filed on May 20, 2003, discloses improvements for adding additional flavor and fragrance to products by inventive packaging that incorporates fragrance-containing material. However, the elimination of off-odors is not completely addressed. Therefore, there remains a long-felt yet unmet need for substantially reducing or eliminating off-odors in foods and other consumer goods. It would thus be desirable to provide materials and methods for packaging foods and other goods that enhance the presence of fragrances. It would further be desirable to provide such improvements in a manner that permitted their application across a wide variety of packaging techniques and that permitted their implementation in a cost-effective manner.

### SUMMARY OF THE INVENTION

Surprisingly, it has now been found that off-odors from foods and other products are efficiently eliminated. The present invention discloses packages with a source of off-odor eliminating compound, a suitable environment for transfer, an appropriate food substrate and a sufficient amount of time for transfer to occur. Examples include a packaging film, packaging tape made from a polymeric material or a paper-based material, a sachet of absorptive material, a granule either by itself or in a polymer matrix, or an active system for delivering off-odor eliminating vapor to the environment via mechanical means. The environment is most preferably closed and of minimum volume in order to provide the maximum concentration of the off-odor controlling substance, such as a sealed food package. However, in certain embodiments within the scope of the present invention, the environment could be more open and provide a flow system in which a product is contacted with an odor controlling vapor. In preferred embodiments, the off-odor eliminating compound is a sulphur scavenger. Materials that were preferably treated using the present invention include snacks such as potato chips, corn chips, cheese flavored products and the like, confections, baked goods, fresh plant materials, cereals and beverages, as well as non-food products. The time required for flavor transfer is dependent primarily on the volatility and concentration of the fragrance, the off-odor eliminating compounds or other out gasses emitted by the products and the packaging, absorptive capacity of the products, and the intensity of the flavor desired.

The present invention thus provides methods of transferring an off-odor eliminating compound to a product comprising the steps of creating a package having an interior and placing a source of the off-odor eliminating compound within the interior and contacting the product with the off-odor eliminating compound. Preferably, the package interior has at least one interior surface containing a source of off-odor eliminating compound, and most preferably, at least a portion of the interior surface is a layer of polymeric film containing the off-odor eliminating compound or a layer of composite of polymeric films and the off-odor eliminating compound. Alternatively, and in accordance with another embodiment of the present invention, a sachet of absorbed off-odor eliminating compound is placed inside the package. In certain other embodiments, this is affected by diffusion through a lid cap or closure is provided for a container that has a compartment that contains an off-odor eliminating compound emitting material or sachet.

In certain preferred embodiments of the present invention the packaging material has at least one polymer layer, and at least one off-odor eliminating compound in an amount sufficient to diffuse within the interior of a package made from the packaging material under normal conditions of temperature and pressure. The packaging material uses either a polymer film containing an off-odor eliminating compound or a polymer film composite of a first polymeric material and an off-odor eliminating compound. In preferred embodiments the material comprises at least an inner layer and an outer barrier layer, with the inner layer containing the off-odor eliminating compound and the outer barrier layer being relatively more inert and a barrier to the diffusion of the off-odor eliminating compound.

The present invention also provides improved product packages that have a polymer impregnated with an off-odor eliminating compound, which is preferably a film laminated to the package, or alternatively is one or more polymer beads, most preferably contained in a sachet. Alternatively, loose polymer strips inserted inside the package may also be used. Off-odor eliminating compound absorbed or otherwise deposited on powdered or granular materials such as silicon dioxide, starch, clay, sugar, salts, cellulose, dextrin, silicate, cellulose, fat, carbon, calcium carbonate, sodium bicarbonate, citric acid, flour, corn meal, and the like may also be packed in a porous sachet for subsequent diffusion into the product when packaged. In still another embodiment, the off-odor eliminating compound can be applied directly to the package contents, or a powder or granular form sprinkled on the product.

Alternatively, in certain preferred embodiments, strips resembling tape or labels may be affixed to an inside surface of the package. The strips contain off-odor eliminating compound, and in certain embodiments will selectively release the off-odor eliminating compound upon the application of an external stimulus, such as heat or pressure. The external stimulus can be related to the process of making the package, the process of using or preparing the package for use, or the process of opening the package. For example, it may be desirable to activate off-odor eliminating compound when sealing a bag during manufacturing, while on the other hand, certain products are improved by releasing off-odor eliminating compound only when the package is heated prior to serving, or opened for serving.

Finally, an alternative embodiment to the closed packages is a package comprising a source of gas flow containing off-odor eliminating compound wherein the off-odor eliminating compound is transferred to the product within the package by the flow of gas contacting the product.

### BRIEF DESCRIPTION OF THE DRAWINGS

**Fig. 1** is a view of a first embodiment of a package made in accordance with the present invention including an expanded view of a cross-section of a sheet of film used in the package.
**Fig. 2** is a cross-sectional view of a second embodiment of a sheet of film made in accordance with the present invention.
**Fig. 3** is a perspective view, partially broken away, of another embodiment of the present invention.
**Fig. 4** is a cross-sectional view of another embodiment of the present invention.
**Fig. 5A** is a plan view of a package made in accordance with the invention in which a label is attached to the side of the package.
**Fig. 5B** is a plan view of an alternative package similar to Fig. 5A.
**Fig. 5C** is a view of an alternative package similar to Fig. 5A.
**Fig. 6A** is a partially broken away plan view of a sealed package made in accordance with the present invention.
**Fig. 6B** is a cross-sectional view of the package shown in Fig. 6A.
**Fig. 7** is a partially broken away plan view of a package made in accordance with the present invention.
**Fig. 8** is a perspective view of a container and lid made in accordance with the present invention.

### DETAILED DESCRIPTION OF THE PREFERRED EMBODIMENTS

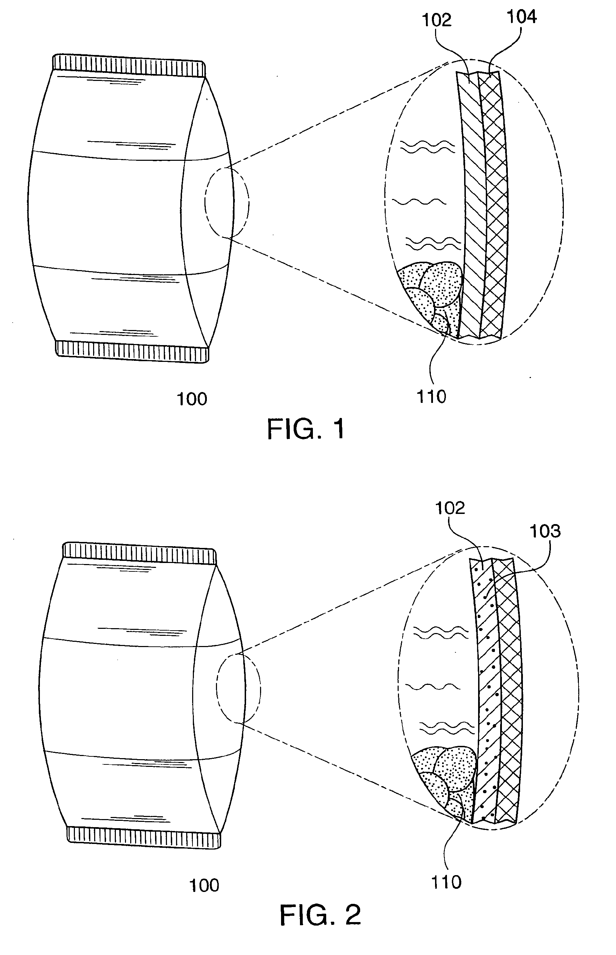

The present invention can be implemented in several preferred embodiments, which are discussed below, along with several illustrative examples. The categories of the invention described below and the examples are provided to provide an understanding of the invention and are not meant to be limiting. In accordance with a first aspect of the present invention, packaging material containing an off-odor eliminating compound is produced using common techniques such as extrusion, film formation and lamination, all well known to those of skill in the art. In a typical embodiment, the off-odor eliminating compound is formed along with the film and a package is produced from the film using conventional techniques. As seen in FIG. 1, a laminated bag 100 comprises a polymeric film layer 102 blended with off-odor eliminating compound and an outer layer 104 made from polymer, foil, or other suitable materials known in the art that act substantially as a barrier to the transmission of the off-odor eliminating compound to the external environment. A product 110 is sealed within the package 100 and over time, the product 110 is in intimate contact with the off-odor eliminating compound (shown by the wavy vapor lines in FIG. 1). Any gas or vapor product present in the package will react with the off-odor eliminating compound that is vaporized within the package 100 (or will transfer it by direct contact). The off-odor eliminating compound is transferred to the product 110 in an amount that is sufficient to substantially reduce or even eliminate off-odors. The technique of making a package from a film material containing the off-odor eliminating compound will be most efficient if the film is used as an inner layer of a packaging laminate that has other layers that are barriers to the diffusion of the off-odor eliminating compound to the environment outside the package, in other words, an impermeable outer layer. This type of packaging could be used on a wide variety of products, including baked goods and snack packages, as well as fruit and vegetable packages.

A second embodiment of the present invention is distinct from the first in that the off-odor eliminating compound is compounded with a polymer to create a composite that diffuses the off-odor eliminating compound into the package. In this embodiment, the end product, such as a packaging bag, can be nearly the same as that discussed previously. The difference lies in that in this particular embodiment the off-odor eliminating compound is non-homogeneously present in the packaging material. As seen in FIG. 2, a package may be constructed substantially in accordance with that described above with reference to FIG. 1, however, the off-odor eliminating compound-containing layer 102 contains off-odor eliminating compound 103 in a matrix. When a package containing at least an element made from such a composite layer 102 is sealed off-odor eliminating compounds are transferred to the product 110. This particular embodiment is widely useful, but would be a particularly preferred implementation for dry drink containers that carry powdered drinks such as instant coffee, iced tea and similar drinks. As in the first embodiment, this embodiment is also preferably used in packaging for snack and baked goods packaging, as well as fresh fruit and vegetable packages.

In either of the embodiments illustrated in FIGS. 1-2, an additional interior layer is added in some embodiments to add strength or to mediate the amount of off-odor eliminating compound transferred over time, by selecting the permeability of the inner layer, e.g., by choosing the sizes of apertures in a perforated sheet. In such embodiments, however, it will be preferable to permit direct contact between the products within the packaging and the off-odor eliminating compound.

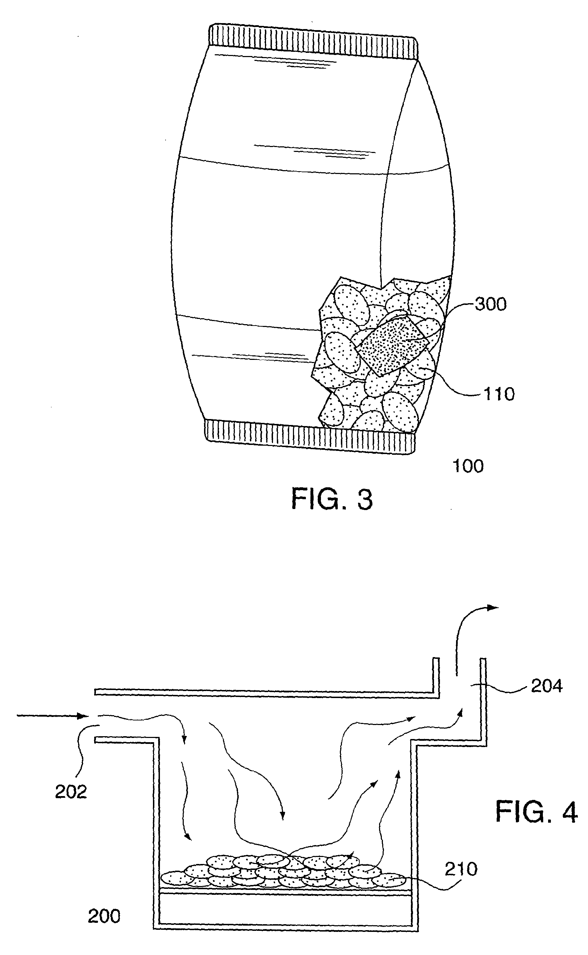

A third embodiment of the present invention is illustrated in FIG. 3, which shows a package 100 substantially the same as that shown in FIGS. 1-2 except that the package contains a sachet 300 as seen in the broken away view. The sachet 300 off-odor eliminating compounds absorbed at high levels on suitable solid supports such as silicon dioxide (SiO₂), clay, dextrin or starch. As seen in FIG. 3, the sachet 300 is disposed within a container or package 100 and emits off-odor eliminating compound while in contact with the product 110 and over time the off-odor eliminating compounds will be transferred from the sachet to the food product or react with gases and vapors emanating from the food product that contain off-odor producing compounds. This embodiment allows the off-odor eliminating element to be made efficiently, and to be added to the food package before or during the packaging operation without affecting the actual construction of the package. In another embodiment, rather than place the powdered or granular off-odor eliminating compounds in a sachet they may be applied directly to the product itself. Similarly, a liquid form of the off-odor eliminating compound can be sprayed directly on to the product, the package or both.

Finally, as mentioned above, in addition to providing a sealed package with a headspace into which a quantity of off-odor eliminating compound will diffuse, it is also feasible to incorporate the present invention into flow through systems that either rely on air circulation or forced ventilation to flow air or other gases over a product while in storage. Such systems can take advantage of the product-enhancing qualities provided by the embodiments described above in situations where a sealed container with a headspace is not practical. Referring now to FIG. 4, there is shown in cross-section a schematic representation of a chamber 200, which may be a portion of a larger apparatus or container, and a source of inlet 202 and exhaust 204. A flow of gas, most preferably air flows through the chamber 200 and contact the product therein 210, as shown by the arrows. The gas includes volatilized off-odor eliminating compound that may be added by any known means. The exhaust may either be directed to another vessel, to the environment or may be recirculated. The exposure of the product to the flow of off-odor eliminating compound containing gas will allow the off-odor eliminating compound to react with any off-odor compounds emanating from the product, as described above with reference to sealed container embodiments. An example of an application of this technique would be during the drying or cooling process for puffed cereals or cookies, where off-odor elimination compound would be desirable, is easily incorporated into the stream of dry air that removes excess moisture and/or cools the product.

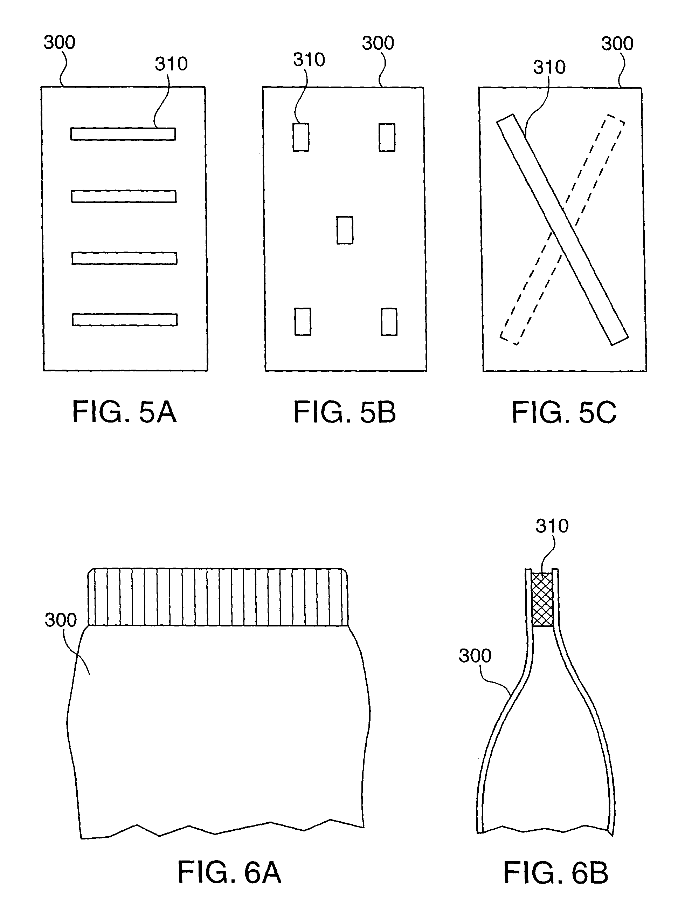

The present invention also provides a number of embodiments by which off-odor eliminating compound is delivered via the material of the package or container in which the materials are not formed as a unitary film. For example, referring to FIGS. 5A-5C, there is shown a plan view of a sheet of material 300 that has one or more strips of thick film 310, for one example a cast film, affixed to one side. The strips 310 are essentially a label or a strip of tape and the technology to manufacture and apply such strips is well known in the plastics, paper-based or cellulose based tape and packaging arts. In the embodiments illustrated in FIGS. 5A-5C the thick film 310 is applied to the package material 300 in a pattern that results in an even distribution of off-odor eliminating compound release within a package. The strips 310 can be arranged in various patterns on one side of the sheet 300, as shown in FIGS. 5A and 5B, or can be applied to both sides, as shown in FIG. 5C. As known in the art, the thick film 310 can be affixed using adhesive, or can be adhered by thermosetting or similar processes that use a combination of heat or pressure to bond layers together.

Referring again to Figure 5A, a label 310 is thus affixed to the material 300. The label is preferably constructed as a sachet-like article affixed to the package, preferably along a package wall, and is preferably affixed to the package wall via an acceptable adhesive, embedded by force, heated to form a weld with the package wall and the like. In another preferred embodiment, the label can be a tape-like material with the off-odor eliminating compound material adhered to one side of the tape and the other side of the tape adhered to the package wall. The size of the label in the package is easily controlled to deliver the desired level of off-odor eliminating compound within the package. A further advantage of this application is the ability to provide multiple labels within the package, as illustrated. The labels are useful for delivering the same off-odor eliminating compound or different off-odor eliminating compounds as well as targeting off-odor eliminating compounds throughout the package 300. For one example, a highly volatile off-odor eliminating compound is provided via a label 310 disposed near the bottom of a package 300, which is then allowed to contact the package contents. Alternatively, a less volatile off-odor eliminating compound could be placed at or near the top of a package and allow the off-odor eliminating compound to disperse to the bottom of the package. In preferred embodiments, the label, tape or thick film 310 will contain off-odor eliminating compound particles blended in a matrix material. The off-odor eliminating compound particles can be absorbed onto a suitable carrier, e.g., food grade acceptable material such as maltodextrin, cornstarch, and the like. In a preferred embodiment the off-odor eliminating compound materials are encapsulated. The encapsulated off-odor eliminating compound materials are placed in a sachet or embedded into a tape, which is placed on the package and the like, as discussed immediately above. An advantage of the encapsulation technology is that it protects the off-odor eliminating compound until activated. For example the capsule material could be compromised by the application of heat, radiation source, pressure and the like.

Referring now to FIGS. 6A-6B, another embodiment of the present invention is shown. In this embodiment, the thick film 310 described above is formulated so that the off-odor eliminating compound is combined with an appropriate polymer or other material that is used as an adhesive (or in conjunction with an adhesive) to seal the package. In accordance with this embodiment of the invention, when the package is opened, off-odor eliminating compound is released. This has the advantage of preventing the contents of the package from absorbing or reacting with the off-odor eliminating compound chemicals during storage but provides the desired release into the headspace during use, which is when off-odors are often created by oxidation. In other words upon breaking the seal, off-odor eliminating compound chemicals are released into the interior of the package and toward the person opening the package, providing the desired effects discussed above. There are numerous encapsulation technologies disclosed in the art.

An alternative embodiment of the present invention provides packages that use well-known and conventional moisture absorbing materials (desiccants) to deliver off-odor eliminating compound to the package contents in the form of a "delivery packet" or sachet, as discussed above. However, instead of being dropped in among the contents of the package, they are adhered to an interior surface of the package. The sachets are attached at strategic locations within the package to provide even distribution. In addition to containing an off-odor eliminating compound, the delivery packet can also contain material that will mask or absorb undesirable compounds that form or are present in the package contents. By this mechanism, the actual shelf life of the product can be extended, or the desirability of the product may be enhanced by eliminating, for example, the perception that a product is "stale" when in fact it is still within its useful shelf life, but has formed reaction products while stored that create the misperception of an adulterated or stale product. For example, 9, 12 - octadecadienoic acid, also known as linolic acid and commercially available as EMERSOL 315, UNIFAC 6550, can be used as a masking agent in the present invention, as discussed above, to absorb sulfur nitrate. Other materials are known that absorb sulphur and other undesirable oxidation compounds frequently found in food and other products. In addition to creating odors, oxidation products often create harmless byproducts that nonetheless mar the product in the package, e.g., white spots. Those of skill in the art can readily formulate additional combinations of materials that absorb undesirable odors, or even react with package contents, to provide enhanced appearance without undue experimentation.

Thus, in accordance with preferred embodiments of this aspect of the present invention, sulphur scavenger compounds are applied directly to the product to remove the sulphur compounds. Sulphur scavenger compound may be included in a wet or dry dough base and will scavenge the sulphur compounds from the ingredients used.

As noted above, sulphur scavenger compounds can also be incorporated into a topical seasoning blend to remove the sulphur compounds from the product and seasoning.

Sulphur scavenger compounds can be included in sweet seasonings and applied to corn/potato or grain based snacks, and sulphur scavenger compounds may also be used in ready to eat products for retail sale that contain sulphur compounds, for example, packaged coleslaw, lettuce. Use of these compounds to remove flavor could result in modifications of flavor perception specifically, but not limited to, corn-based snack products. Certain regions of the world do not care for the strong flavor of corn but like the majority of the snack characteristics. This scavenger allows for the use of a low price ingredient such as corn while delivering a consumer acceptable snack.

Sulphur scavenger compounds will also allow delivering sweet seasonings for historically savory products or deliver historically difficult flavor combinations with corn based snacks. As noted above, in addition to enhanced product characteristics, the present invention provides the advantage of extended shelf along with extended flavor intensity and/or quality.

As discussed above with reference to FIGS. 6A-6B, the sachets used in certain embodiments of the present invention are preferably formulated with "capsules" that release off-odor eliminating compound upon the application of mechanical pressure or other force. This has the advantage of minimizing exposure to (or loss of) the off-odor eliminating compound material during process of manufacturing the final product, for example, filling a package with food. Contamination of both the environment and the off-odor eliminating compound material itself is thereby reduced. Thus, in one aspect of these embodiments of the present invention, a preferred method of manufacturing a package is provided wherein pressure-activated capsules are packaged with sachets, and this combination is added to a package, either by being dropped in, or preferably adhered to the interior of the package as discussed above.

The package is then sealed, and in the sealing process, rollers or other devices place mechanical force upon the package at the location of the sachets. The force breaks the capsules so the off-odor eliminating compound is released into the sealed container.

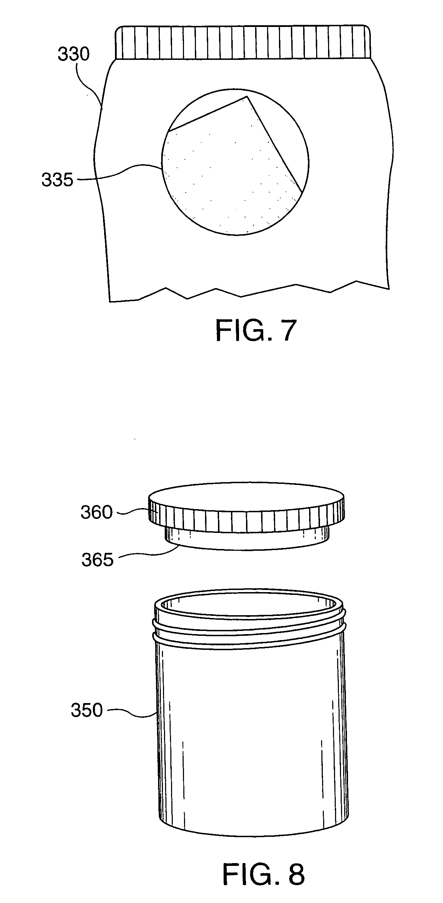

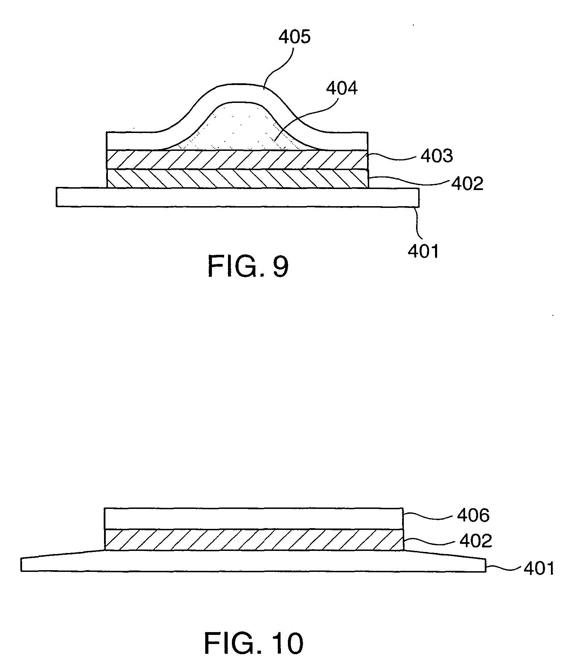

Alternatively, in certain embodiments of the present invention, off-odor eliminating compound can be impregnated into paper or similar support materials, such as woven or non-woven fabrics. The impregnated section of the material is coated such that the off-odor eliminating compound is activated by a trigger such as heat, moisture or mechanical force. One example of this embodiment of the present invention is illustrated in FIG. 7. In this example, a paper or paper laminate bag 330 has an impregnated section 335 that an off-odor eliminating compound that is formulated so that it is released upon melting, i.e., upon application of heat to the product during preparation. As known in the art, different materials absorb microwave radiation at different rates, and thus provide differential heating patterns that can be used advantageously with the present invention. Numerous types of food products (and some non-food products) are heated in both microwave and conventional ovens, for both individual and institutional use, and would be improved by incorporating this aspect of the present invention. Such applications of the invention are not limited to impregnated paper containers, since many packaged food items are provided in aluminum or metallized paper or polymer film containers. It is preferred, however, to provide at least one layer of material, whether woven, non-woven, polymeric, paper-based or otherwise that is impregnated with the desired off-odor eliminating compound.

Referring to FIG. 8, yet another embodiment of the present invention is illustrated. In this embodiment, the off-odor eliminating compound is to a flavor carrier that is integrated into a lid, cap or closure 360 that is used in conjunction with a container 350. Preferably, a compartment 365 is provided as part of the lid 360. In certain embodiments, the packets or sachets discussed above are simply disposed within the compartment 365, which is designed to be air permeable, and thus permits the off-odor eliminating compound or flavor in the sachet to be admitted to the headspace above the contents of the container 350, either continuously, or upon the application of force during opening of the container, or upon the application of heat or other stimulus, as discussed above. Thus, for one example, the lid 360 could be for a single serving soup package, and upon heating in a microwave, the materials disposed in the compartment would emit scents that complimented or enhanced the soup yet would become diminished or adulterated if mixed with the soup itself.

In any preferred embodiment of the present invention the off-odor eliminating compound containing material, or at least the vapors emanating therefrom, in any form, is in contact with the contents of the package. Thus, as explained above, it is preferred that in certain embodiments where an interior barrier layer is incorporated for strength, aesthetic or other reasons, this layer will most preferably contain perforations or apertures so as to allow contact between the off odor eliminating compound containing layer and package contents. However, it is also preferred that the packaging is substantially air impermeable and holds the off-odor eliminating compound in the head space above the product, typically requiring an additional container element or at the very least a laminated film with an air-impermeable outer layer. In certain preferred embodiments, the packaging is resealable, as is well known in the art using a "zipper" feature or the like in the case of a pouch or bag, or in other embodiments, a cap or lid. After closing the resealable device the headspace over the product is refilled with the off-odor eliminating compound chemical thus providing a renewed concentration of off-odor eliminating compound that is released and sensed by the consumer when the container is reopened.

In these preparations, the compound of the present invention can be used alone or in combination with other off-odor eliminating compound compositions, solvents, adjuvants and the like. Those with skill in the art will appreciate the nature and variety of the other ingredients that can be used in combination with the compound of the present invention.

Many types of off-odor eliminating compounds can be employed in the present invention, the only limitation being the compatibility with the other components being employed and the suitability to include the off-odor eliminating compound with a food when used in this manner. Suitable off-odor eliminating compounds include but are not limited sulphur scavenging compounds. One preferred material is a spraydried mixture of C16-C18 acids, predominately the C16 acid, linoleic acid; available from International Flavors & Fragrances, New York, N.Y. This material can contain a variety of other related compounds including palmitoleic acid, linolelaidic acid, linoelaidic acid, isolinoleic acid, trans, trans, trans-9,12,15-octadecatrienoic acid, and the like. By preponderance, it is understood that more than about 50 weight percent, preferably more than about 60 and most preferably more than about 75 weight percent of the sulfur scavenging material is the identified compound.

This material is usually dissolved in oil. In preferred embodiments, the scavenger is applied at 0.5% topically or in topical seasoning. When compounded in a polymeric material, the concentration may vary from about 0.1 to about 30, preferably from about 2 to about 20 weight percent in oil, preferably in low density polyethylene (LDPE). In general, the level of an off-odor eliminating compound that would preserve a product's olfactory profile varies from about 0.005 to about 20 weight percent and is likely from about 0.5 to about 5 weight percent. Those of skill in the art will understand, therefore, that reduction below these levels would be desirable to significantly reduce or eliminate the possibility of detecting the off-odor. In addition to the compounds, other agents can be used in conjunction with the off-odor eliminating compound. Well known materials such as surfactants, emulsifiers, and polymers to encapsulate the off-odor eliminating compound are employed without departing from the scope of the present invention.

As used herein "olfactory effective amount" is understood to mean the amount of compound in compositions the individual component will contribute to its particular olfactory characteristics, but the olfactory effect of the composition will be the sum of the effects of each of the ingredients. Thus, the compounds of the invention can be used to alter the aroma characteristics by modifying the olfactory reaction contributed by another ingredient in the composition. The amount will vary depending on many factors including other ingredients, their relative amounts and the effect that is desired.

The term "food" or "foodstuff' as used herein includes both solid and liquid ingestible materials for man or animals, which materials usually do, but need not, have nutritional value. Thus, foodstuffs include meats, gravies, soups, convenience foods, malt, alcoholic and other beverages, milk and dairy products, seafoods, including fish, crustaceans, mollusks and the like, candies, vegetables, cereals, soft drinks, snacks, dog and cat foods, other veterinary products and the like.

When the compounds of this invention are used, they can be combined with conventional flavoring materials or adjuvants. Such co-ingredients or flavor adjuvants are well known in the art for such use and have been extensively described in the literature. Requirements of such adjuvant materials are that they be ingestibly acceptable and thus nontoxic or otherwise non-deleterious. Apart from these requirements, conventional materials can be used and broadly include other flavor materials, vehicles, stabilizers, thickeners, surface active agents, conditioners and flavor intensifiers. Note that materials that have low volatility are added directly to the food product as they will not be transferred via vapor diffusion in an efficient manner. For example, salts and other taste active materials may be added directly to potato chips as is normal practice, while volatile flavors are transferred to the product via the vapor phase from a suitable source placed within the package.

Such conventional flavoring materials include saturated fatty acids, unsaturated fatty acids and amino acids; alcohols including primary and secondary alcohols, esters, carbonyl compounds including ketones and aldehydes; lactones; other cyclic organic materials including benzene derivatives, alicyclic compounds, heterocyclics such as furans, pyridines, pyrazines and the like; sulfur-containing compounds including thiols, sulfides, disulfides and the like; proteins; lipids, carbohydrates; so-called flavor potentiators such as monosodium glutamate; magnesium glutamate, calcium glutamate, guanylates and inosinates; natural flavoring materials such as cocoa, vanilla and caramel; essential oils and extracts such as anise oil, clove oil and the like and artificial flavoring materials such as vanillin, ethyl vanillin and the like. Off-odor eliminating compounds useful in the various embodiments of the present invention will cover a similarly wide range of chemical compositions, although as noted above, the prevalence of sulphur compounds in several known off odors results in sulfur scavenging compounds being among the preferred embodiments of the present invention.

The off-odor eliminating compounds can be combined with one or more vehicles or carriers for adding them to the particular product. Vehicles can be edible or otherwise suitable materials such as ethyl alcohol, propylene glycol, water and the like, as described supra. Carriers include materials such as gum arabic, carrageenan, xanthan gum, guar gum and the like.

A particularly effective method to carry out the present invention is to incorporate the off-odor eliminating compound in a polymer that is the contact layer in the invention. A preferred embodiment is POLYIFF polymer products produced by International Flavors & Fragrances Inc., New York, N.Y. These polymers have been formed in fabrics as disclosed in U.S. Patent 6,500,444; in sachets as described in U.S. Patent 6,213,409, and in fiber form as set forth in U.S. Patent 6,207,274. The polymer can be formed into the appropriate form using extrusion, calendaring film-forming or other appropriate technology to create the polymer shape desired. Suitable polymers include but are not limited to polyethylene, including linear low density, low density, and copolymers of polyethylene, polypropylene, polystyrene, ester terminated polyamide, polyethylene terephlalate and polystyrene. Alternatively, the off-odor eliminating compound can be incorporated in a wax or fat material, preferably a lining material that contains a wax or fat material.

Those of skill in the art will understand that the time required for flavor and/or off-odor eliminating compound transfer is dependent primarily on the volatility and concentration of the off-odor eliminating compounds, absorptive capacity of the food, and the intensity of the flavor desired. Additionally, as relative concentrations change over time, the equilibrium of the off-odor eliminating compound within a package will shift, particularly for longer shelf life items. However, the concentrations and techniques used to implement the present invention over any time period are well within the grasp of those of skill in the art and will not require undue experimentation to determine the optimal construction of a package made in accordance with the present invention.

All U.S. patents and patent applications referenced in this application are hereby incorporated by reference. Upon review of the foregoing, numerous adaptations, modifications, and alterations will occur to the reviewer. These will all be, however, within the spirit of the present invention. Accordingly, reference should be made to the appended claims in order to ascertain the true scope of the present invention. The following examples are presented to further describe and enable the invention without limiting the invention to the examples presented. All percents are understood to be weight percent unless noted to the contrary.

### EXAMPLE 1

This example demonstrates that the scavenger sulfur compound was effective in minimizing the characteristic corn flavor from several snack foods.

The flavor characteristic of various snack products: such as corn chips, ranch flavored corn chips, nacho cheese tortilla chips, and baked corn chips purchased at a supermarket were modified by sealing each of these products into a sleeve of film that was infused with scavenger, the predominately C16 acid, linoleic acid described above. The film strips were prepared at two usage levels in the product, 1% and 2% loading in the film strips.

Both levels of the scavenger were evaluated for each of the products listed above. For each product, 50 grams of each product were placed in an 18 inch sleeve of film and sealed in an 8 inch by 12 inch foil (metal) bag.

After storing these products for 24 hours, the corn based snacks were evaluated by taste and smell compared to a control (no scavenger film). The volatile odors associated with the typical corn characteristics of corn chips and tortilla chips were minimized using the scavenger materials compared to the control that did not employ the scavenger materials.

Assessment of nacho cheese tortilla chips and ranch flavor corn chips indicated the onion, garlic and corn flavor were minimized in the scavenged packaging as compared to the control product. Baked tortilla chips showed the least effect with minimal change in flavor as compared to the control materials.

Also included in this trial, potato chips and sour cream and onion flavored potato chips. The traditional (salted only) chips had a more astringent, drying character but only minimally so. The sour cream and onion flavored potato chips were perceived to be more dairy-like with less onion than the control.

### EXAMPLE 2

Due to the success of the sulfur scavenging compound in the above example when infused into the packaging, the idea to include this material onto chips was initiated. Spray dried versions of the sulfur scavenger, the predominately C 16 acid, linoleic acid described above, were prepared and tested on snack products. Various levels were tested including 0.5%, 0.75%, 1% and 1.5% of the sulfur scavenger on the snack product. Based on taste testing performed by the chemists, the most effective level was 0.5% on chips based on the reduced impression of corn flavor.

Further testing involved using the sulfur scavenger materials with a topical seasoning. This test evaluated a cheese flavor ranch seasoning flavor with 0.5 weight % scavenger, the predominately C16 acid, linoleic acid described above, and coated onto the corn chips. When compared to control, the product with scavenger had a more dairy, buttery flavor with less corn character. Strong aromas are common characteristics of certain snack foods. In some cases, this is considered a negative attribute and an effort to reduce, modify or eliminate the strong aroma is desirable as was accomplished by use of the sulfur scavenging materials of the present invention.

## Claims

1. A method of transferring an off-odor eliminating compound to a product comprising the steps of: creating a package having an interior; placing a source of said off-odor eliminating compound within said interior; and contacting the product with said source.

2. The method of claim 1 wherein said package interior has at least one interior surface, and said surface contains an off-odor eliminating compound.

3. The method of claim 2 further comprising the step of constructing at least a portion of said interior surface from a layer of polymeric film containing said off-odor eliminating compound.

4. The method of claim 2 further comprising the step of applying a strip to said interior surface wherein the strip comprises said off-odor eliminating compound.

5. The method of claim 1, wherein said source comprises off-odor eliminating compound applied directly to the package.

6. The method of claim 1, wherein said source comprises a sachet of off-odor eliminating compound.

7. The method of claim 1, wherein the off-odor eliminating compound is a sulphur scavenging material.

8. A packaging material comprising at least one packaging material layer and at least one off-odor eliminating compound in an amount sufficient to diffuse within the interior of a package made from the packaging material under normal conditions of temperature and pressure.

9. The packaging material of claim 8, wherein at least one packaging material layer is a polymer layer comprising a polymer film containing a off-odor eliminating compound.

10. The packaging material of claim 8, wherein at least one polymer layer comprises a polymer film composite of a first polymeric material and an off-odor eliminating compound.

11. The packaging material of claim 8 wherein the packaging material comprises at least an inner layer and an outer barrier layer, said inner layer containing said off-odor eliminating compound and said outer barrier layer being relatively more inert and relatively air impermeable to create barrier to diffusion of said off-odor eliminating compound to an external environment.

12. The packaging material of claim 11, wherein the inner layer comprises a material impregnated with an off-odor eliminating compound.

13. The packaging material of claim 11, wherein the inner layer comprises a material impregnated with a sulphur scavenging material.

14. The packaging material of claim 12, wherein the material is paper.

15. The packaging material of claim 12, wherein the off-odor eliminating compound is formulated to release off-odor eliminating compound upon the application of an external stimulus.

16. The packaging material of claim 15 wherein the external stimulus is chosen from the group consisting of heat, mechanical energy and microwave energy.

17. The packaging material of claim 8 wherein said material is produced by one of the following methods: extrusion lamination, film formation.

18. The packaging material of claim 8 wherein the off-odor eliminating compound is applied to the packaging material as a powder.

19. The packaging material of claim 8 wherein the off-odor eliminating compound is applied to the packaging material as a liquid.

20. A product package comprising a package, and a product contained within the package, and further comprising a material impregnated with an off-odor eliminating compound in contact with said product.

21. The product package of claim 20, wherein the material is a film laminated to the package.

22. The product package of claim 20, wherein the material comprises one or more granular elements.

23. The product package of claim 22, wherein said granular elements are contained in a sachet.

24. The product package of claim 22, wherein the granular elements are comprised of a polymer and an off-odor eliminating compound.

25. The product package of claim 24, wherein the polymer is selected from the group consisting of ethyl-vinyl acetate, polyethylene, polypropylene, polystyrene, ester terminated polyamide, polyethylene terephlalate or polystyrene.

26. The product package of claim 22, wherein the granular elements are comprised of:
(a) a substrate chosen from the group consisting essentially of silicon dioxide, starch, clay, sugar, salts, cellulose, dextrin, silicate, cellulose, fat, carbon, calcium carbonate, sodium bicarbonate, citric acid, flour, or corn meal; and
(b) an off-odor eliminating compound.

27. The product package of claim 20, wherein the material comprises loose polymer strips inserted inside the package.

28. The product package of claim 27, wherein the polymer strips are comprised of ethyl-vinyl acetate.

29. The product package of claim 20, wherein the material comprises at least one strip affixed to an inner surface of the package.

30. The product package of claim 29, wherein the strip is affixed to a package seal.

31. The package of claim 20 wherein the off-odor eliminating compound is a sulphur scavenger.

32. The package of claim 20 wherein the product is edible.

33. The package of claim 20 wherein the package comprises a resealable pouch.

34. The package of claim 20 wherein the package comprises a rigid, resealable container and a closure.

35. The package of claim 34, wherein the closure further comprises a compartment and the compartment contains the material impregnated with an off-odor eliminating compound.

36. The package of claim 20, wherein the material impregnated with an off-odor eliminating compound comprises a material that releases the off-odor eliminating compound upon an external stimulus.

37. The package of claim 33, wherein the external stimulus is one or more chosen from the group consisting of heat pressure and microwave radiation.

38. Apparatus for adding an off-odor eliminating compound to a product comprising a source of gas flow containing an off-odor eliminating compound wherein the off-odor eliminating compound is transferred to a product within the chamber by contacting the product with the gas flow.

39. The apparatus of claim 38, wherein the apparatus comprises an outlet.

40. The apparatus of claim 38, wherein the outlet directs a stream of outlet gas to be recalculated.

41. A consumer product comprising a sulphur scavenging compound topically applied to the consumer product.

42. The consumer product of claim 41, wherein the consumer product is a foodstuff.

43. The consumer product of claim 41, wherein the sulphur scavenging compound is applied at about 0.5 % weight percent.
